(19) Europäisches Patentamt
European Patent Office
Office européen des brevets

(11) Veröffentlichungsnummer: **0 173 258**
**A2**

(12) **EUROPÄISCHE PATENTANMELDUNG**

(21) Anmeldenummer: **85110577.5**

(22) Anmeldetag: **22.08.85**

(51) Int. Cl.4: **C07D 405/06**, A61K 31/41, A61K 31/415, C07D 295/08, C07D 317/22

(30) Priorität: **31.08.84 CH 4177/84**

(43) Veröffentlichungstag der Anmeldung: **05.03.86 Patentblatt 86/10**

(84) Benannte Vertragsstaaten: **AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Kompis, Ivan, Dr.**
**Goldentalweg 16**
**CH-4104 Oberwil(CH)**
Erfinder: **Weiss, Ekkehard, Dr.**
**Unterer Baselblick 21B**
**D-7851 Inzlingen(DE)**

(74) Vertreter: **Lederer, Franz, Dr. et al**
**Patentanwälte Dr. Lederer Franz Meyer-Roxlau Reiner F. Lucile-Grahn-Strasse 22**
**D-8000 München 80(DE)**

(54) **Substituierte 2-Phenyl-1,3-dioxolane.**

(57) Die neuen Verbindungen der Formel

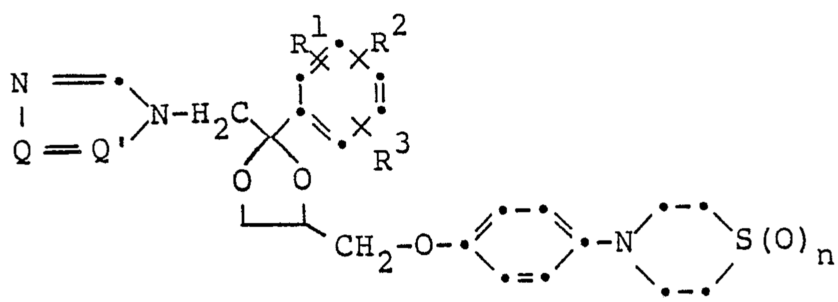

worin eines der Symbole Q und Q' die Gruppe -CH= und das andere die Gruppe -CH= oder -N=, $R^1$, $R^2$ und $R^3$ je Wasserstoff oder Halogen und n die Zahl 0, 1 oder 2 bedeuten, und ihre pharmazeutisch annehmbaren Säureadditionssalze besitzen wertvolle antimykotische Eigenschaften und können zur Bekämpfung von Infektionen verwendet werden, welche durch pathogene Pilze hervorgerufen werden.

EP 0 173 258 A2

Substituierte 2-Phenyl-1,3-dioxolane

Die vorliegende Erfindung betrifft neue 2-Phenyl-1,3-dioxolane der allgemeinen Formel

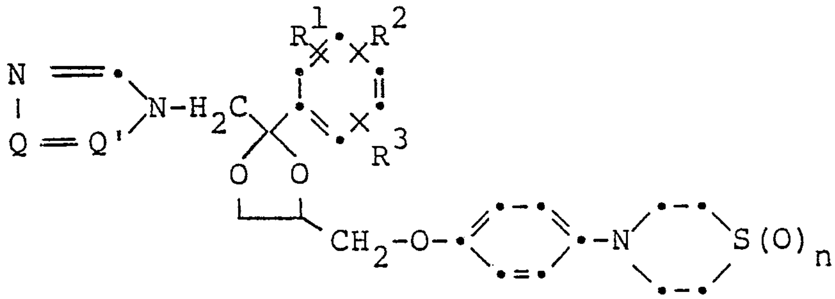

worin eines der Symbole Q und Q' die Gruppe -CH= und das andere die Gruppe -CH= oder -N=, $R^1$, $R^2$ und $R^3$ je Wasserstoff oder Halogen und n die Zahl 0, 1 oder 2 bedeuten, und pharmazeutisch annehmbare Säureadditionssalze davon.

Diese Verbindungen besitzen wertvolle antimykotische Eigenschaften und können zur Bekämpfung von Infektionen verwendet werden, welche durch pathogene Pilze hervorge rufen werden.

Die vorliegende Erfindung betrifft: Die neuen Verbindungen der Formel 1 und ihre pharmazeutisch annehmbaren Säureadditionssalze als solche und zur Anwendung als therapeutische Wirkstoffe, Verfahren und Zwischenprodukte zu deren Herstellung, diese enthaltende Arzneimittel und deren Herstellung, sowie deren Verwendung bei der Bekämpfung oder Verhütung von Krankheiten und zur Herstellung von antimykotisch wirksamen Mitteln.

Der Ausdruck "Halogen" bezeichnet die vier Formen Chlor, Brom, Fluor und Jod. Der weiter unten verwendete Ausdruck "Abgangsgruppe" bezeichnet durch Substitution leicht austauschbare Atome oder Gruppen, beispielsweise Halogenatome, wie Chlor, Brom und Jod, niedere Alkylsulfonyloxygruppen, wie die Methansulfonyloxygruppe, und Arylsulfonyloxygruppen, wie die p-Toluolsulfonyloxygruppe. In diesem Zusammenhang bevorzugte Arylgruppen sind gegebenenfalls durch niederes Alkyl oder Halogen substituierte Phenylgruppen. Der Ausdruck "niederes Alkyl" bezeichnet geradkettige oder verzweigte gesättigte Kohlenwasserstoffreste mit bis zu 7, vorzugsweise bis zu 4 Kohlenstoffatomen, wie Methyl und dgl.

In einer bevorzugten Ausführungsform bedeuten Q und Q' beide die Gruppe -CH=. Vorzugsweise bedeuten $R^1$ und $R^2$ je Halogen und $R^3$ Wasserstoff. In einer ganz besonders bevorzugten Ausführungsform bedeuten $R^1$ und $R^2$ beide Chlor und $R^3$ Wasserstoff, und $R^1$ und $R^2$ befinden sich in der 2- bzw. 4-Stellung. Vorzugsweise bedeutet n die Zahl 2.

Eine besonders bevorzugte Verbindung der Formel I ist:

cis-4-[4-[[2-(2,4-Dichlorphenyl)-2-[(imidazol-1-yl)-methyl]-1,3-dioxolan-4-yl]methoxy]phenyl]-tetrahydro-4H-1,4-thiazin-1,1-dioxid.

Weitere spezifische Vertreter der durch die allgemeine Formel I definierten Stoffklasse sind:

cis-4-[4-[[2-(2,4-Dichlorphenyl)-2-[(imidazol-1-yl)-methyl]-1,3-dioxolan-4-yl]methoxy]phenyl]-tetrahydro-4H-1,4-thiazin,

cis-4-[4-[[2-(2,4-Dichlorphenyl)-2-[(imidazol-1-yl)-methyl]-1,3-dioxolan-4-yl]methoxy]phenyl]-tetrahydro-4H-1,4-thiazin-1-oxid,

cis-4-[4-[[2-(2,4-Dichlorphenyl)-2-[(1H-1,2,4-triazol-1--yl)methyl]-1,3-dioxolan-4-yl]methoxy]phenyl]-tetrahydro-4H--1,4-thiazin,

cis-4-[4-[[2-(2,4-Dichlorphenyl)-2-[(1H-1,2,4-triazol-1--yl)methyl]-1,3-dioxolan-4-yl]methoxy]phenyl]-tetrahydro-4H--1,4-thiazin-1-oxid,

cis-4-[4-[[2-(2,4-Dichlorphenyl)-2-[(1H-1,2,4-triazol-1--yl)methyl]-1,3-dioxolan-4-yl]methoxy]phenyl]-tetrahydro-4H--1,4-thiazin-1,1-dioxid und

cis-4-[4-[[2-(2,4-Dichlorphenyl)-2-[(4H-1,2,4-triazol-4--yl)methyl]-1,3-dioxolan-4-yl]methoxy]phenyl]-tetrahydro-4H--1,4-thiazin-1,1-dioxid.

Die Verbindungen der Formel I und ihre pharmazeutisch annehmbaren Säureadditionssalze können dadurch hergestellt, dass man

a) eine Verbindung der allgemeinen Formel

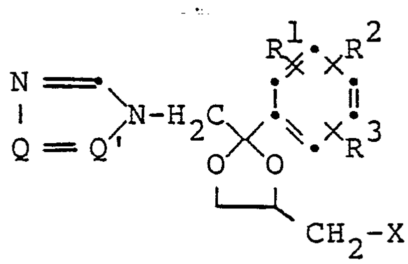

worin Q, Q', $R^1$, $R^2$ und $R^3$ obige Bedeutung besitzen und X eine Abgangsgruppe bedeutet, in Gegenwart einer Base mit einer Verbindung der allgemeinen Formel

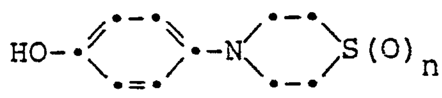

worin n obige Bedeutung besitzt, umsetzt; oder

b) eine Verbindung der allgemeinen Formel

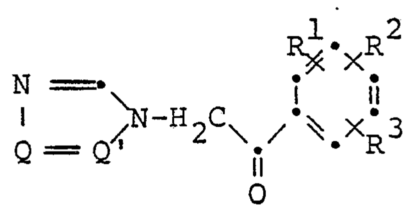

worin Q, Q', $R^1$, $R^2$ und $R^3$ obige Bedeutung besitzen, mit einem Glykol der allgemeinen Formel

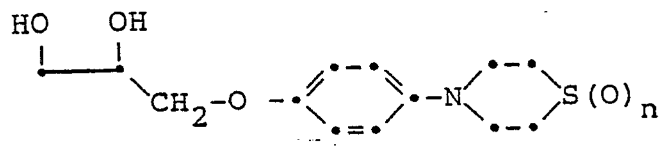

worin n obige Bedeutung besitzt, in das entsprechende Ketal überführt, oder

c) eine Verbindung der allgemeinen Formel

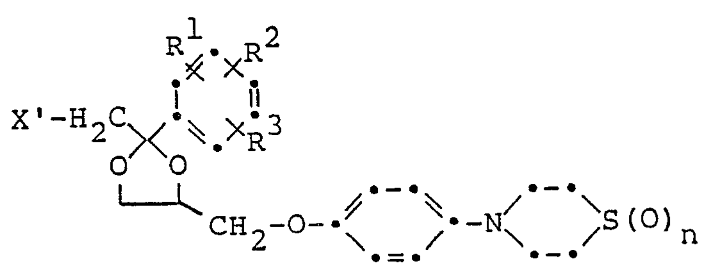

worin $R^1$, $R^2$, $R^3$ und n obige Bedeutung besitzen und X' eine Abgangsgruppe bedeutet, mit einer Verbindung der allgemeinen Formel

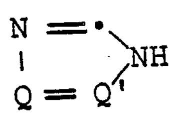

worin Q und Q' obige Bedeutung besitzen, umsetzt, oder

d) eine Verbindung der allgemeinen Formel

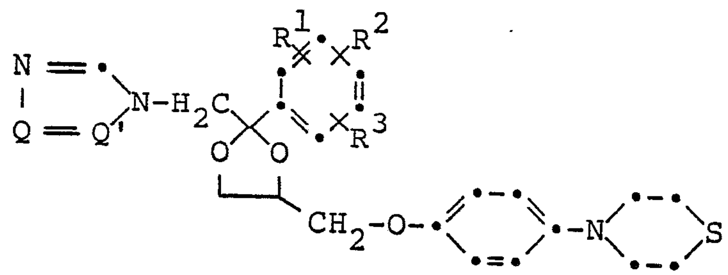

worin R¹, R², R³, Q und Q' obige Bedeutung besitzen, am Schwefelatom oxidiert, und

e) erwünschtenfalls eine Verbindung der Formel I in ein pharmazeutisch annehmbares Säureadditionssalz überführt.

Die Reaktion einer Verbindung der Formel II mit einem Phenol der Formel III gemäss Verfahrensvariante a) wird vorzugsweise in N,N-Dimethylformamid, Dimethylsulfoxid oder in einem inerten Aether, wie Tetrahydrofuran, Aethylenglykoldimethyläther und t-Butylmethyläther durchgeführt. Die mit X bezeichnete Abgangsgruppe ist vorzugsweise eine niedere Alkylsulfonyloxy- oder eine Arylsulfonyloxygruppe. Eine ganz besonders bevorzugte Abgangsgruppe ist die Methansulfonyloxygruppe. Geeignete Basen sind beispielsweise Natriumhydrid, Alkalimetallhydroxide, wie Natriumhydroxid und Kaliumhydroxid, und Alkalimetallcarbonate, wie Kaliumcarbonat. In einer besonders bevorzugten Ausführungs form verwendet man als Base Natriumhydrid, das in Mineralöl dispergiert ist. Die Reaktion kann in einem Temperaturbereich von etwa 20°C bis etwa 100°C durchgeführt werden.

Die Reaktion eines Ketons der Formel IV mit einem Glykol der Formel V zum entsprechenden Ketal gemäss Verfahrensvariante b) wird vorzugsweise in Gegenwart einer starken Säure, wie p-Toluolsulfonsäure, und unter azeotroper Entfernung des gebildeten Reaktionswassers durchgeführt. Geeignete Lösungsmittel sind beispielsweise aromatische Kohlenwasserstoffe, wie Benzol, Toluol und Xylol, halogenierte Kohlenwasserstoffe, wie Methylenchlorid und Chloroform, sowie Mischungen dieser Lösungsmittel mit niederen Alkoholen, wie Aethanol, Propanol, Butanol oder Pentanol. Die Umsetzung erfolgt bei der Siedetemperatur des gewählten Lösungsmittels bzw. bei der Siedetemperatur des gewählten Lösungsmittelgemisches.

Die Reaktion einer Verbindung der Formel VI mit einer Verbindung der Formel VII gemäss Verfahrensvariante c) wird vorzugsweise in einem inerten organischen Lösungsmittel, wie N,N-Dimethylformamid, Dimethylsulfoxid oder N,N-Dimethylacetamid, in Gegenwart einer Base durchgeführt. Geeignete Basen sind beispielsweise Alkalimetallcarbonate, wie Natrium- und Kaliumcarbonat, Alkalimetallalkoholate, wie Natrium- und Kaliummethanolat, tertiäre Amine, wie Tri- äthylamin, Natriumhydrid und dgl. Als Base kann jedoch auch die Verbindung der Formel VII verwendet werden, welche dann natürlich im Ueberschuss eingesetzt werden muss. Die mit X' bezeichnete Abgangsgruppe ist vorzugsweise ein Halogenatom, beispielsweise ein Chlor-, Brom- oder Jodatom. Die Reaktion wird zweckmässigerweise in einem Temperaturbereich von etwa 0°C bis etwa 170°C durchgeführt.

Die Oxidation einer Verbindung der Formel Ia zu einer Verbindung der Formel I, worin n die Zahl 1 bedeutet, gemäss Verfahrensvariante d) wird vorzugsweise mit einer Per säure, wie m-Chlorperbenzoesäure, in einem inerten organischen Lösungsmittel bewerkstelligt. Geeignete Lösungsmittel sind beispielsweise halogenierte Kohlenwasserstoffe, wie Dichlormethan und Chloroform. Die Reaktion wird vorzugsweise in einem Temperaturbereich von etwa -20°C bis Raumtemperatur durchgeführt.

Die Verbindungen der Formel I können in pharmazeutisch annehmbare Säureadditionssalze übergeführt werden. Die Herstellung von solchen Säureadditionssalzen erfolgt dabei nach an sich bekannten und jedem Fachmann geläufigen Methoden. Es kommen sowohl Salze mit pharmazeutisch annehmbaren anorganischen als auch Salze mit pharmazeutisch annehmbaren organischen Säuren in Betracht, beispielsweise Hydrochloride, Hydrobromide, Sulfate, Nitrate, Citrate, Acetate, Succinate, Methansulfonate, p-Toluolsulfonate und dgl.

Die als Ausgangsstoffe verwendeten Verbindungen der Formel II, worin Q die Gruppe -N= und Q' die Gruppe -CH= bedeuten, sind ebenfalls neu und können hergestellt werden, indem man in einer Verbindung der allgemeinen Formel

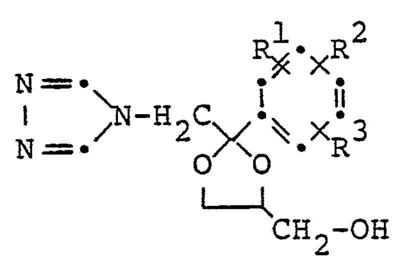

worin R¹, R² und R³ obige Bedeutung besitzen, die Hydroxylgruppe in an sich bekannter Weise durch eine Abgangsgruppe ersetzt bzw. in eine solche überführt. Die niederen Alkylsulfonate und die Arylsulfonate können beispielsweise durch Behandeln einer Verbindung der Formel VIII mit einem entsprechenden Sulfonsäurechlorid in Gegenwart einer Base, wie Pyridin, hergestellt werden. Die Verbindungen der Formel VIII und die brigen Verbindungen der Formel II sind bekannte Substanzen.

Die als Ausgangsstoffe verwendeten Verbindungen der Formel III, worin n die Zahl 2 bedeutet, sind neu und können hergestellt werden, indem man 4-Aminophenol in einem niederen Alkohol, wie Aethanol, mit Divinylsulfon umsetzt. Diese Reaktion wird vorzugsweise in einem Temperaturbereich von etwa Raumtemperatur bis zum Siedepunkt der Reaktionsmischung durchgeführt. Die übrigen Verbindungen der Formel III sind bekannte Substanzen.

Die als Ausgangsstoffe verwendeten Verbindungen der Formeln V und VI sind neu und können beispielsweise gemäss dem nachfolgenden Reaktionsschema I hergestellt werden.

Reaktionsschema I

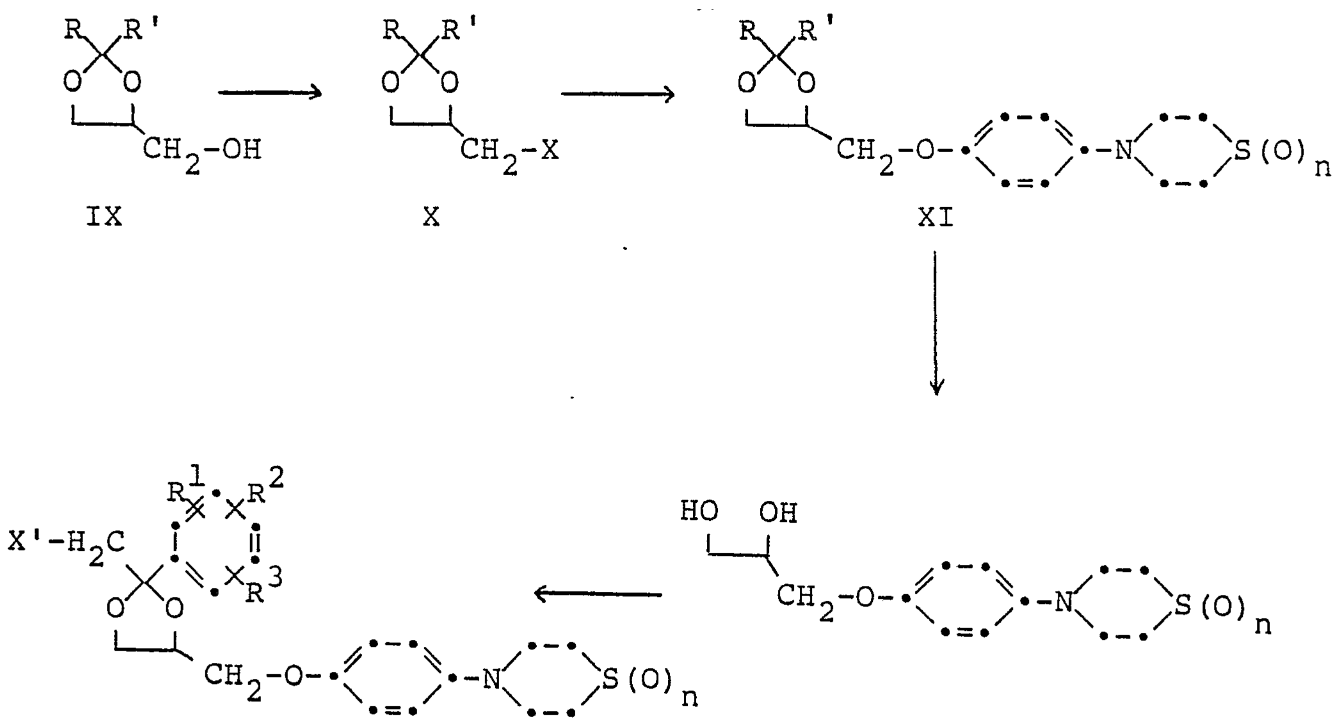

IX X XI

$R^1$, $R^2$, $R^3$, X, X' und n besitzen obige Bedeutung; R und R' bedeuten je Wasserstoff oder niederes Alkyl.

Die Verbindungen der Formel X können aus den bekannten Verbindungen der Formel IX hergestellt werden, und zwar in Analogie zur weiter oben beschriebenen Herstellung von Ver bindungen der Formel II, worin Q die Gruppe -N= und Q' die Gruppe -CH= bedeuten, aus Verbindungen der Formel VIII. Die Herstellung einer Verbindung der Formel XI aus einer Verbindung der Formel X erfolgt in Analogie zur Herstellung von Verbindungen der Formel I aus Verbindungen der Formel II. Die Ueberführung einer Verbindung der Formel XI in eine Verbindung der Formel V kann beispielsweise durch Hydrolyse des 1,3-Dioxolanringes in Gegenwart von Wasser und einer starken Säure, wie p-Toluolsulfonsäure, oder durch Umacetalisieren in Gegenwart eines niederen Alkohols, wie Methanol, und einer Säure, wie p-Toluolsulfonsäure, bewerkstelligt werden.

Die Herstellung von Verbindungen der Formel VI aus Verbindungen der Formel V erfolgt in Analogie zur Herstellung von Verbindungen der Formel I aus Verbindungen der Formel V, und zwar durch Umsetzen einer Verbindung der Formel V mit einer Verbindung der allgemeinen Formel

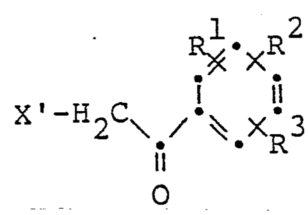

worin $R^1$, $R^2$, $R^3$ und X' obige Bedeutung besitzen.

Die als Ausgangsstoffe verwendeten Verbindungen der Formeln IX und XII gehören an sich bekannten Substanzklassen an.

Die Verbindungen der Formel II, worin Q die Gruppe -N= und Q' die Gruppe -CH= bedeuten, die Verbindungen der Formel III, worin n die Zahl 2 bedeutet, sowie die Verbindungen der Formeln V und VI sind ebenfalls Gegenstand der vorliegenden Erfindung.

Wie bereits erwähnt besitzen die Verbindungen der Formel I und ihre pharmazeutisch annehmbaren Säureadditions salze wertvolle antimykotische Eigenschaften. Sie sind wirksam gegen eine Vielzahl von pathogenen Pilzen, die topische und systemische Infektionen hervorrufen. Sie sind beispielsweise wirksam gegen Candida albicans und Histoplasma capsulatum, was beispielsweise wie folgt gezeigt werden kann:

Der Test zur Prüfung auf fungistatische Aktivität wird in Teströhrchen durchgeführt, die jeweils 4,5 ml Casitonagar enthalten und während 15 Minuten bei 120°C sterilisiert worden sind. Die zu untersuchenden Verbindungen werden in einer Konzentration von 20 mg/ml in Dimethylsulfoxid gelöst, worauf man die Lösungen mit sterilem, destilliertem Wasser auf eine Konzentration von 10 mg/ml verdünnt. Anschliessend wird eine Serie von Lösungen bereitet, indem man die ursprüngliche Lösung durch Versetzen mit sterilem, destilliertem Wasser nacheinander jeweils auf ein Zehntel der ursprünglichen Konzentration verdünnt. Zu jedem Röhrchen, das 4,5 ml Casitonagar enthält, werden dann jeweils 0,5 ml Vorratslsung zugegeben, wobei Wirkstoffkonzentrationen von 100, 10, 1 oder 0,1 µg/ml Medium erhalten werden.

Anschliessend zählt man Zellen von Candida albicans (Kulturen, welche 24 Stunden alt sind) aus und gibt jeweils $10^3$ Zellen pro ml in die vorbereiteten Testrhörchen (50°C), welche dann schräg gelegt werden. Die Zellen von Histoplasma capsulatum werden in einer Konzentration von ca. $10^7$ Hefezellen pro ml in die Teströhrchen gegeben, wobei man in diesem Fall Kulturen verwendet, welche 3 Tage alt sind. Im Falle von Candida albicans bebrütet man während 48 Stunden bei 37°C, und im Falle von Histoplasma capsulatum bebrütet man während 7 Tagen bei 30°C. Die MIC-Werte werden also nach 48 Stunden bzw. nach 7 Tagen ermittelt. Die so ermittelten Ergebnisse sind in der nachfolgenden Tabelle I zusammengestellt. Diese Tabelle enthält ausserdem noch Angaben über die akute Toxizität der erfin dungsgemässen Verbindungen ($DL_{50}$ nach einmaliger oraler Verabreichung an Mäusen):

Tabelle I

| Q | Q' | n | 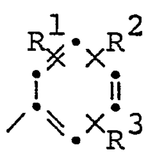 | MIC-Werte in µg/ml H.capsulatum | C.albicans | $DL_{50}$ in mg/kg |
|---|---|---|---|---|---|---|
| -CH= | -CH= | 0 | 2,4-Dichlor-phenyl | 10 | 1 -100 | ca. 1000 |
| -CH= | -CH= | 1 | " | 10 | 1 -100 | ca. 1000 |
| -CH= | -CH= | 2 | " | 1 | 0,1-1 | ≥ 4000 |
| -CH= | -N= | 0 | " | 10 | 0,1-1 | ≥ 5000 |
| -CH= | -N= | 1 | " | 100 | 1 -100 | ca. 4000 |
| -CH= | -N= | 2 | " | 1 | 0,1-10 | ≥ 5000 |
| -N= | -CH= | 2 | " | >100 | 0,1-10 | ≥ 4000 |

Die erfindungsgemässen Produkte können in Form pharmazeutischer Präparate als Heilmittel Verwendung finden. Die pharmazeutischen Präparate können enteral, z.B. in Form von Tabletten, Lacktabletten, Dragées, Hart- und Weichgelatinekapseln, Lösungen, Emulsionen, Suspensionen oder Suppositorien, parenteral, z.B. in Form von Injektionslösungen, oder topisch, z.B. in Form von Salben, Crèmen oder Gelen, verabreicht werden.

Zur Herstellung solcher Präparate können die erfindungsgemässen Produkte mit pharmazeutisch inerten, anorganischen oder organischen Trägern verarbeitet werden. Geeignete Träger sind beispielsweise Lactose, Maisstärke oder Derivate davon, Talk, Stearinsäure oder deren Salze, pflanzliche Oele, Wachse, Fette, halbfeste und flüssige Polyole, Wasser, Saccharose, Invertzucker, Glucose und dgl.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten. Die Herstellung solcher pharmazeutischer Präparate erfolgt nach an sich bekannten und jedem Fachmann geläufigen Methoden.

Wie bereits erwähnt, sind solche pharmazeutischen Präparate und deren Herstellung ebenfalls Gegenstand der vorliegenden Erfindung.

Die erfindungsgemässen Produkte können bei der Bekämpfung bzw. Verhütung von Krankheiten, insbesondere bei der Bekämpfung pathogener Pilze verwendet werden. Die Dosierung kann dabei innerhalb weiter Grenzen variieren und ist natürlich in jedem einzelnen Fall den individuellen Gegebenheiten anzupassen. Je nach Applikationsart werden erfindungsgemässe Substanzen in einer täglichen Dosierung von etwa 0,5 mg/kg bis etwa 200 mg/kg verabreicht.

Die nachfolgenden Beispiele dienen der näheren Erläuterung der vorliegenden Erfindung. Sie sollen den Umfang derselben jedoch in keiner Weise beschränken. Alle Temperaturen sind in Celsiusgraden angegeben.

## Beispiel 1

aa) Ein Gemisch aus 99 g Hydrochinon, 13,5 g Kupfer(I)chlorid und 180 ml Thiomorpholin wird unter Argon während 47 Stunden unter Rückfluss zum Sieden erhitzt. Nach dem Abkühlen verteilt man zwischen 1N-Natronlauge und Dichlormethan, stellt die Natronlaugeauszüge unter Eiskühlung mit konz. Salzsäure auf etwa pH 1 und verteilt dann zwischen 0,1N-Salzsäure und Dichlormethan, das 10% Alkohol enthält.

Die sauren Auszüge werden unter Eiskühlung mit 50-proz. Natronlauge auf etwa pH 6 gestellt und danach je dreimal mit Dichlormethan, das 10% Alkohol enthält, sowie Dichlormethan, das 20% Alkohol enthält, extrahiert. Das erhaltene Material wird im Kugelrohr bei 180° und 0,01 Torr destilliert und dann aus Methanol/Aether umkristallisiert. Man erhält p-(Tetrahydro-4H-1,4-thiazin-4-yl)phenol vom Schmelzpunkt 156-158°.

ab) Zu 17,17 g p-Fluornitrobenzol werden bei ca. 80° 23 ml Thiomorpholin langsam so zugetropft, dass die Innentemperatur nicht über 110° steigt. Dann wird noch während 1 Stunde bei 120° gerührt, worauf man dreimal mit je 1 1 Dichlormethan extrahiert und die organischen Phasen dreimal mit je 500 ml Wasser wäscht, über Natriumsulfat trocknet und eindampft. Durch Umkristallisieren des Rohproduktes aus Dichlormethan/Aether erhält man 4-(p-Nitrophenyl)-tetrahydro-4H-1,4-thiazin vom Schmelzpunkt 142-144°.

Eine Lösung von 23,47 g 4-(p-Nitrophenyl)-tetrahydro-4H-1,4-thiazin in 1,5 1 Dioxan wird über Raneynickel hydriert, bis die Wasserstoffaufnahme beendet ist. Danach filtriert man vom Katalysator ab, wäscht gut mit Dioxan und dampft das Filtrat im Vakuum ein. Durch Umkristallisieren des Rohproduktes aus Dichlormethan/Aether erhält man 4-(p-Aminophenyl)-tetrahydro-4H-1,4-thiazin vom Schmelzpunkt 109-111°.

Man löst 2,34 g 4-(p-Aminophenyl)-tetrahydro-4H-1,4-thiazin in 12 ml 35-proz. Schwefelsäure, gibt 12 g Eis dazu, leitet dann langsam eine kalte Lösung von 1,05 g Natriumnitrit in 12 ml Wasser unter die Oberfläche und rührt während 5 Minuten bei etwa 0°. Danach werden 60 mg Harnstoff, eine Lösung von 144 g Kupfer(II)nitrat-trihydrat in 420 ml Wasser und schliesslich unter starkem Rühren 1,59 g Kupfer(I)oxid zugegeben. Man rührt während 5 Minuten, stellt mit Natronlauge auf etwa pH 6 und extrahiert dreimal mit je 600 ml Dichlormethan und einmal mit 600 ml Dichlormethan/Aethanol (4:1). Die organischen Phasen werden zweimal mit je 300 ml Wasser gewaschen, über Natriumsulfat getrocknet und eingedampft. Das Rohprodukt wird an 400 g Kieselgel mit Dichlormethan, das 5% Aceton enthält, chromatographiert. Das erhaltene Material wird aus Dichlormethan/Methanol/Aether umkristallisiert, wobei man p-(Tetrahydro-4H-1,4-thiazin-4-yl)phenol vom Schmelzpunkt 155-157° erhält.

b) Zu einem Gemisch aus 815 mg Natriumhydrid-Dispersion (55%) in Oel und 27ml N,N-Dimethylformamid wird unter Argon eine Lösung von 2,69 g p-(Tetrahydro-4H-1,4-thiazin-4-yl)phenol in 54 ml N,N-Dimethylformamid zugetropft, worauf man während 30 Minuten bei 40° rührt. Danach wird bei 20° eine Lösung von 5,09 g cis-[2-(2,4-Dichlorphenyl) - 2-[(1H-imidazol-1-yl)methyl] - 1,3-dioxolan-4-yl]methylmethansulfonat in 102 ml N,N-Dimethylformamid zugetropft. Man rührt während 2,5 Stunden bei 90°, gibt unter Eiskühlung 150 ml Wasser dazu und extrahiert dreimal mit je 300 ml Dichlormethan. Die organischen Phasen werden zweimal mit je 150 ml Wasser gewaschen, über Natriumsulfat getrocknet und im Vakuum eingedampft. Der Rückstand wird aus Dichlormethan/Aether umkristallisiert. Man erhält cis-4-[4-[[2-(2,4-Dichlorphenyl) -2-[(imidazol-1-yl)methyl] - 1,3-dioxolan-4-yl]methoxy]phenyl]tetrahydro -4H-1,4-thiazin vom Schmelzpunkt 164-166°.

## Beispiel 2

Man löst 1,56 g cis-4-[4-[[2-(2,4-Dichlorphenyl) - 2-[(imidazol-1-yl)methyl] - 1,3-dioxolan-4-yl]methoxy]-phenyl]-tetrahydro - 4H-1,4-thiazin in 30 ml Dichlormethan, gibt 600 mg Kaliumcarbonat dazu, kühlt auf 0° ab, tropft langsam 17,5 ml einer 4,2-proz. Lösung von m-Chlorperbenzoesäure in Dichlormethan/Aethylacetat (1:1) dazu und rührt dann noch während 30 Minuten bei 0°. Nach Zugabe von 75 ml Wasser und 300 mg Natriumsulfit wird dreimal mit je 150 ml Dichlormethan extrahiert. Die organischen Phasen werden mit 10-proz. Kaliumbicarbonatlösung und Wasser gewaschen, über Natriumsulfat getrocknet und im Vakuum eingedampft. Das erhaltene Rohprodukt wird an 390 g Kieselgel unter Eluieren mit Dichlormethan, das 5% Methanol enthält, chromatographiert. Durch Umkristallisieren aus Aethanol/Aether erhält man cis-4-[4-[[2-(2,4-Dichlorphenyl) -2-[(imidazol-1-yl)-methyl] - 1,3-dioxolan-4-yl]methoxy]phenyl]-tetrahydro - 4H-1,4-thiazin-1-oxid vom Schmelzpunkt 174-175°.

## Beispiel 3

a) Zu 11,34 g Divinylsulfon wird bei 50° eine Suspension von 8,74 g 4-Aminophenol in 190 ml Aethanol zugetropft, worauf man noch mit 50 ml Aethanol spült und dann unter Argon während 3 Stunden unter Rückfluss zum Sieden erhitzt. Nach dem Abkühlen versetzt man mit 480 ml Wasser und rührt während 1 Stunde bei 0°. Die ausgefallenen Kristalle werden abgenutscht, mit 20-proz. Aethanol. und Wasser gewaschen und getrocknet. Durch Umkristallisieren aus Dichlormethan/Methanol/Aether erhält man 4-(p-Hydroxyphenyl)-tetrahydro-4H-1,4-thiazin-1,1-dioxid vom Schmelzpunkt 153-155°.

b) Zu einem Gemisch aus 920 mg Natriumhydrid-Dispersion (55%) in Oel und 26 ml N,N-Dimethylformamid wird eine Lösung von 3,08 g 4-(p-Hydroxyphenyl)-tetrahydro-4H-1,4-thiazin-1,1-dioxid in 62 ml N,N-Dimethylformamid zugetropft, worauf man während 15 Minuten bei 40° unter Argon rührt. Danach tropft man bei 20° eine Lösung von 4,93 g cis-[2-(2,4-Dichlorphenyl) -2-[(1H-imidazol-1-yl)methyl] - 1,3-dioxolan-4-yl]methylmethansulfonat in 99 ml N,N-Dimethylformamid dazu, rührt unter Argon während 2.5 Stunden bei 90°, versetzt unter Eiskühlung mit 150 ml

Wasser und extrahiert dreimal mit je 300 ml Dichlormethan. Die organischen Phasen werden zweimal mit je 150 ml Wasser gewaschen, über Natriumsulfat getrocknet und eingedampft. Das Rohpro dukt wird aus Dichlormethan/Aether kristallisiert und aus Aethanol umkristallisiert. Man erhält cis-4-[4-[[2-(2,4-Dichlorphenyl) -2-[(imidazol-1-yl)methyl] - 1,3-dioxolan-4-yl]methoxy]phenyl]-tetrahydro - 4H-1,4-thiazin-1,1-dioxid vom Schmelzpunkt 170-172°.

Beispiel 4

Zu einem Gemisch aus 1,02 g Natriumhydrid-Dispersion (55%) in Oel und 34 ml N,N-Dimethylformamid wird eine Lösung von 3,37 g p-(Tetrahydro-4H-1,4-thiazin-4-yl)phenol in 68 ml N,N-Dimethylformamid zugetropft, worauf man während 30 Minuten bei 40° unter Argon rührt. Danach tropft man bei 20° eine Lösung von 6,38 g cis-[[2-(2,4-Dichlorphenyl) -2-[(1H-1,2,4-triazol-1-yl)methyl] - 1,3-dioxolan-4-yl]methyl]methansulfonat in 128 ml N,N-Dimethylformamid dazu, rührt unter Argon während 2,5 Stunden bei 90°, versetzt unter Eiskühlung mit 200 ml Wasser und extrahiert dreimal mit je 400 ml Dichlormethan und einmal mit Dichlormethan/Aethanol (4:1). Die organischen Phasen werden nacheinander zweimal mit je 200 ml Wasser gewaschen, über Natriumsulfat getrocknet und im Vakuum eingedampft. Das kristalline Rohprodukt wird aus Dichlormethan/Aether umkristallisiert. Man erhält farbloses cis-4-[4-[[2-(2,4--Dichlorphenyl) - 2-[(1H-1,2,4-triazol-1-yl)methyl] - 1,3-dioxolan-4-yl]methoxy]phenyl]-tetrahydro -4H-1,4-thiazin vom Schmelzpunkt 119-121°.

Beispiel 5

Zu einem Gemisch aus 3,41 g cis-4-[4-[[2-(2,4-Dichlorphenyl) - 2-[(1H-1,2,4-triazol-1-yl)methyl] - 1,3-dioxolan-4-yl]methoxy]phenyl]-tetrahydro - 4H-1,4-thiazin, 1,47 g Kaliumcarbonat und 130 ml Dichlormethan wird bei 0° eine Lösung von 1.63 g 90-proz. m-Chlorperbenzoesäure in 80 ml Dichlormethan langsam und unter starkem Rühren zugetropft. Nach weiteren 30 Minuten bei 0° werden 320 ml Wasser und 1,14 g Natriumsulfit dazugegeben, worauf man dreimal mit je 320 ml Dichlormethan extrahiert. Die organischen Phasen werden einmal mit 160 ml 10-proz. Kaliumbicarbonatlösung und zweimal mit je 160 ml Wasser gewaschen, über Natriumsulfat getrocknet und eingedampft. Das erhaltene Rohprodukt wird an 650 g Kieselgel mit Dichlormethan, das 2-5% Methanol enthält, chromatographiert. Durch Kristallisieren des erhaltenen Materials aus Dichlormethan/Aether und Umkristallisieren aus Aethanol/Aether erhält man cis-4-[4-[[2-(2,4-Dichlorphenyl) - 2-[(1H-1,2,4-triazol-1-yl)methyl] - 1,3-dioxolan-4-yl]methoxy]phenyl]-tetrahydro - 4H-1,4-thiazin-1-oxid vom Schmelzpunkt 125-129°.

Beispiel 6

In Analogie zu Beispiel 3b) erhält man aus 4-(p-Hydroxyphenyl)-tetrahydro-4H-1,4-thiazin-1,1-dioxid und cis-[[2-(2,4-Dichlorphenyl) - 2-[(1H-1,2,4-triazol-1-yl)-methyl] - 1,3-dioxolan-4-yl]methyl]methansulfonat das cis-4-[4-[[2-(2,4-Dichlorphenyl) - 2-[(1H-1,2,4-triazol-1-yl)methyl] - 1,3-dioxolan-4-yl]methoxy]phenyl]-tetrahydro - 4H-1,4-thiazin-1,1-dioxid vom Schmelzpunkt 142-144°.

Beispiel 7

a) Man löst 2,04 g cis-[2-(2,4-Dichlorphenyl) - 2-[(4H-1,2,4-triazol-4-yl)methyl] -1,3-dioxolan]-4-methanol vom Schmelzpunkt 181-182° in 10 ml Pyridin, tropft bei 0° 1,13 g Methansulfochlorid dazu, rührt während 15 Minuten bei 20° und während 2,5 Stunden bei 30°, versetzt dann bei 0° mit 100 ml Wasser und rührt während 45 Minuten bei 0°. Die ausgefallenen Kristalle werden abgenutscht, mit Wasser gewaschen und im Vakuum getrocknet. Durch Umkristallisieren aus Dichlormethan/Aether erhält man cis-[[2-(2,4-Dichlorphenyl) -2-[(4H-1,2,4-triazol-4-yl)methyl] - 1,3-dioxolan-4-yl]methyl]methansulfonat vom Schmelzpunkt 135-137°.

b) Zu einem Gemisch aus 540 mg Natriumhydrid-Dispersion (55%) in Oel und 12 ml N,N-Dimethylformamid wird bei 20° eine Lösung von 1,41 g 4-(p-Hydroxyphenyl)-tetrahydro-4H-1,4-thiazin-1,1-dioxid in 28 ml N,N-Dimethylformamid getropft, worauf man unter Argon während 15 Minuten bei 40° rührt. Dann wird bei 20° eine Lösung von 2,25 g cis-[[2-(2,4-Dichlorphenyl) -2-[(4H-1,2,4-triazol-4-yl)methyl] - 1,3-dioxolan-4-yl]methyl]methansulfonat in 45 ml N,N-Dimethylformamid dazugetropft und unter Argon während 2,5 Stunden bei 90° gerührt. Unter Eiskühlung werden danach 75 ml Wasser zugetropft, worauf man dreimal mit je 150 ml Dichlormethan extrahiert. Die organischen Phasen werden zweimal mit je 75 ml Wasser gewaschen, über Natriumsulfat getrocknet und im Vakuum eingedampft. Das erhaltene Rohprodukt wird an 660 g Kieselgel mit Dichlormethan, das 2-4% Methanol enthält, chromatographiert. Durch Kristallisieren aus Dichlormethan/Aether und Umkristallisieren aus Aethanol/Aether erhält man cis-4-[4-[[2-(2,4-Dichlorphenyl) - 2-[(4H-1,2,4-triazol-4-yl)methyl] - 1,3-dioxolan-4-yl]-methoxy]phenyl]-tetrahydro - 4H-1,4-thiazin-1,1-dioxid vom Schmelzpunkt 202-206°.

Beispiel A

Die Verbindung cis-4-[4-[[2-(2,4-Dichlorphenyl) - 2-[(imidazol-1-yl)methyl] - 1,3-dioxolan-4-yl]methoxy]-phenyl]-tetrahydro - 4H-1,4-thiazin-1,1-dioxid kann wie folgt als Wirkstoff zur Herstellung von Tabletten verwendet werden:

| Bestandteile | mg/Tablette |
|---|---|
| Wirkstoff | 200 |
| Milchzucker pulv. | 100 |
| Polyvinylpyrrolidone | 15 |
| Na-carboxymethylstärke | 10 |
| Talk | 3 |
| Magnesiumstearat | 2 |
| Tablettengewicht | 330 |

Der Wirkstoff und der Milchzucker pulv. werden intensiv gemischt. Die erhaltene Mischung wird dann mit einer wässrigen Lösung von Polyvinylpyrrolidone befeuchtet und geknetet, worauf man die erhaltene Masse granuliert, trocknet und siebt. Man mischt das Granulat mit den übrigen Bestandteilen und verpresst dann zu Tabletten geeigneter Grösse.

**Ansprüche**

1. Verbindungen der allgemeinen Formel

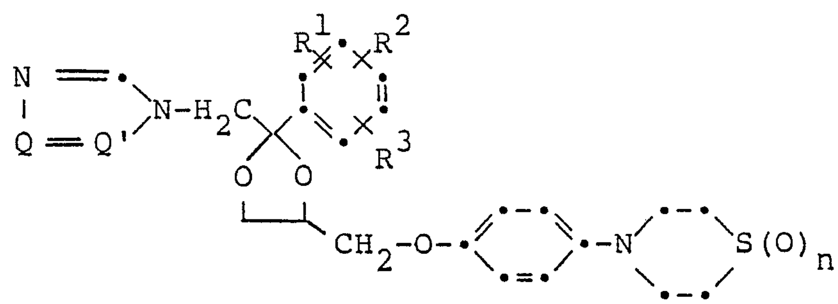

worin eines der Symbole Q und Q' die Gruppe -CH= und das andere die Gruppe -CH= oder -N=, $R^1$, $R^2$ und $R^3$ je Wasserstoff oder Halogen und n die Zahl 0, 1 oder 2 bedeuten, und pharmazeutisch annehmbare Säureadditionssalze davon.

2. Verbindungen nach Anspruch 1, worin Q und Q' beide die Gruppe -CH= bedeuten.

3. Verbindungen nach Anspruch 1 oder 2, worin $R^1$ und $R^2$ je Halogen und $R^3$ Wasserstoff bedeuten.

4. Verbindungen nach Anspruch 3, worin $R^1$ und $R^2$ sich in der 2- bzw. 4-Stellung befinden und beide Chlor bedeuten.

5. Verbindungen nach einem der Ansprüche 1 bis 4, worin n die Zahl 2 bedeutet.

6. cis-4-[4-[[2-(2,4-Dichlorphenyl)-2-[(imidazol-1-yl)-methyl]-1,3-dioxolan-4-yl]methoxy]phenyl]-tetrahydro-4H-1,4-thiazin-1,1-dioxid.

7. cis-4-[4-[[2-(2,4-Dichlorphenyl) -2-[(imidazol-1-yl)methyl]-1,3-dioxolan-4-yl]methoxy]phenyl]-tetrahydro-4H-1,4-thiazin, cis-4-[4-[[2-(2,4-Dichlorphenyl) -2-[(imidazol-1-yl)methyl]-1,3-dioxolan-4-yl]methoxy]phenyl]-tetrahydro-4H-1,4-thiazin-1-oxid, cis-4-[4-[[2-(2,4-Dichlorphenyl)-2-[(1H-1,2,4-triazol-1-yl)methyl]-1,3-dioxolan-4-yl]methoxy]phenyl]-tetrahydro-4H-1,4-thiazin, cis-4-[4-[[2-(2,4-Dichlorphenyl)-2-[(1H-1,2,4-triazol-1-yl)-methyl]-1,3-dioxolan-4-yl]methoxy]phenyl]-tetrahydro-4H-1,4-thiazin-1-oxid, cis-4-[4-[[2-(2,4-Dichlorphenyl)-2-[(1H-1,2,4-triazol-1-yl)methyl]-1,3-dioxolan-4-yl]-methoxy]phenyl]-tetrahydro-4H-1,4-thiazin-1,1-dioxid und cis-4-[4-[[2-(2,4-Dichlorphenyl)-2-[(4H-1,2,4-triazol-4-yl)methyl]-1,3-dioxolan-4-yl]methoxy]phenyl]-tetrahydro-4H-1,4-thiazin-1,1-dioxid.

8. 4-(p-Hydroxyphenyl)-tetrahydro-4H-1,4-thiazin-1,1-dioxid.

9. Verbindungen der allgemeinen Formel

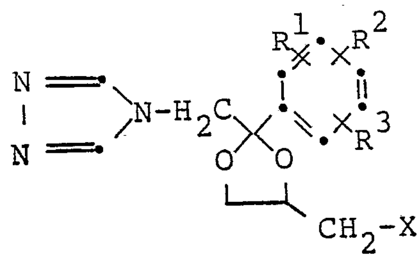

worin $R^1$, $R^2$und $R^3$ je Wasserstoff oder Halogen und X eine Abgangsgruppe bedeuten.

10. Verbindungen der allgemeinen Formel

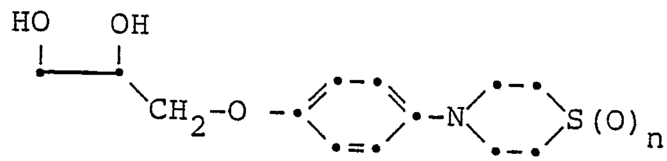

worin n die Zahl O, 1 oder 2 bedeutet.

11. Verbindungen der allgemeinen Formel

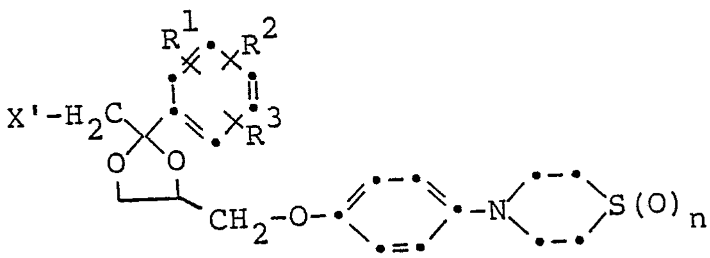

worin $R^1$, $R^2$und $R^3$ je Wasserstoff oder Halogen, X' eine Abgangsgruppe und n die Zahl O, 1 oder 2 bedeuten.

12. Verbindungen nach einem der Ansprüche 1 bis 7 zur Anwendung als therapeutische Wirkstoffe.

13. Verbindungen nach einem der Ansprüche 1 bis 7 zur Anwendung als antimykotische Wirkstoffe.

14. Verfahren zur Herstellung von Verbindungen nach einem der Ansprche 1 bis 7, dadurch gekennzeichnet, dass man

a) eine Verbindung der allgemeinen Formel

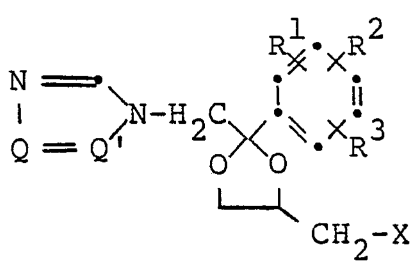

worin Q, Q', $R^1$, $R^2$ und $R^3$ die in Anspruch 1 angegebene Bedeutung besitzen und X eine Abgangsgruppe bedeutet, in Gegenwart einer Base mit einer Verbindung der allgemeinen Formel

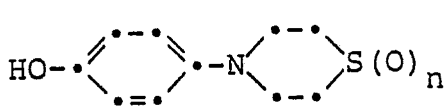

worin n die in Anspruch 1 angegebene Bedeutung besitzt, umsetzt, oder

b) eine Verbindung der allgemeinen Formel

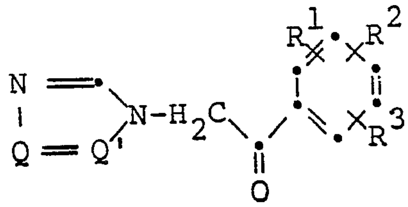

worin Q, Q', $R^1$, $R^2$ und $R^3$ die in Anspruch 1 angegebene Bedeutung besitzen, mit einem Glykol der allgemeinen Formel

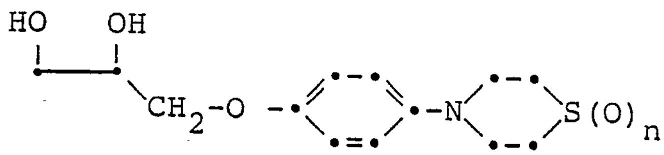

worin n die in Anspruch 1 angegebene Bedeutung besitzt, in das entsprechende Ketal überführt, oder

c) eine Verbindung der allgemeinen Formel

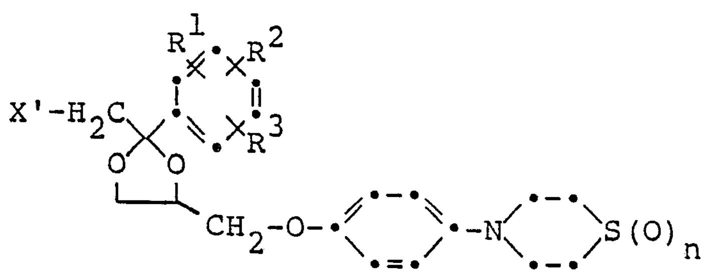

worin $R^1$, $R^2$, $R^3$ und n die in Anspruch 1 angegebene Bedeutung besitzen und X' eine Abgangsgruppe bedeutet, mit einer Verbindung der allgemeinen Formel

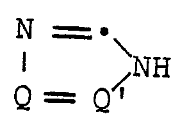

worin Q und Q' die in Anspruch 1 angegebene Bedeutung besitzen, umsetzt, oder

d) eine Verbindung der allgemeinen Formel

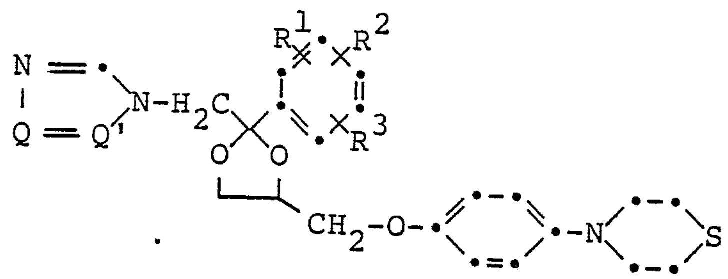

worin $R^1$, $R^2$, $R^3$, Q und Q' die in Anspruch 1 angegebene Bedeutung besitzen, am Schwefelatom oxidiert, und

e) erwünschtenfalls eine Verbindung der Formel I in ein pharmazeutisch annehmbares Säureadditionssalz überführt.

15. Arzneimittel enthaltend eine Verbindung nach einem der Ansprüche 1 bis 7 und ein therapeutisch inertes Trägermaterial.

16. Antimykotisch wirksame Mittel enthaltend eine Verbindung nach einem der Ansprüche 1 bis 7 und ein therapeutisch inertes Trägermaterial.

17. Verwendung von Verbindungen nach einem der Ansprüche 1 bis 7 bei der Bekämpfung oder Verhütung von Krankheiten.

18. Verwendung von Verbindungen nach einem der Ansprüche 1 bis 7 bei der Bekämpfung von pathogenen Pilzen.

19. Verwendung von Verbindungen nach einem der Ansprüche 1 bis 7 zur Herstellung von antimykotisch wirksamen Mitteln.

Patentansprüche für den Vertragsstaat: AT

1. Verfahren zur Herstellung von Verbindungen der allgemeinen Formel

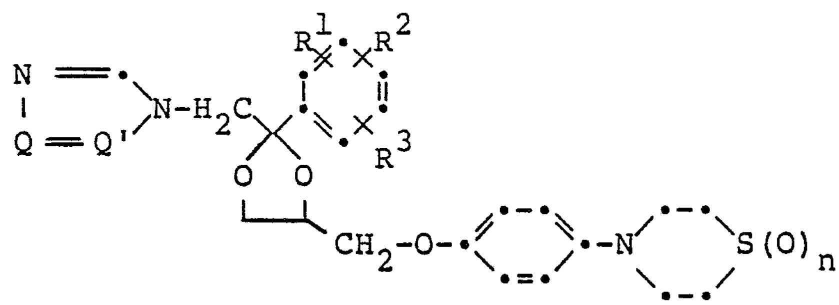

worin eines der Symbole Q und Q' die Gruppe -CH= und das andere die Gruppe -CH= oder -N=, $R^1$, $R^2$ und $R^3$ je Wasserstoff oder Halogen und n die Zahl 0, 1 oder 2 bedeuten, und pharmazeutisch annehmbaren Säureadditionssalzen davon, dadurch gekennzeichnet, dass man

a) eine Verbindung der allgemeinen Formel

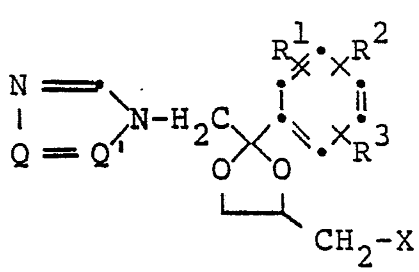

worin Q, Q', $R^1$, $R^2$ und $R^3$ obige Bedeutung besitzen und X eine Abgangsgruppe bedeutet, in Gegenwart einer Base mit einer Verbindung der allgemeinen Formel

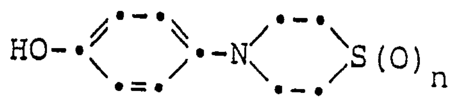

worin n obige Bedeutung besitzt, umsetzt, oder

b) eine Verbindung der allgemeinen Formel

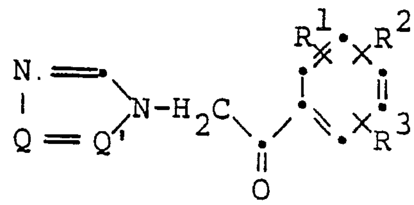

worin Q, Q', $R^1$ , $R^2$ und $R^3$ obige Bedeutung besitzen, mit einem Glykol der allgemeinen Formel

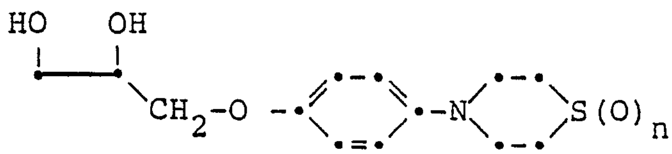

worin n obige Bedeutung besitzt, in das entsprechende Ketal überführt, oder

c) eine Verbindung der allgemeinen Formel

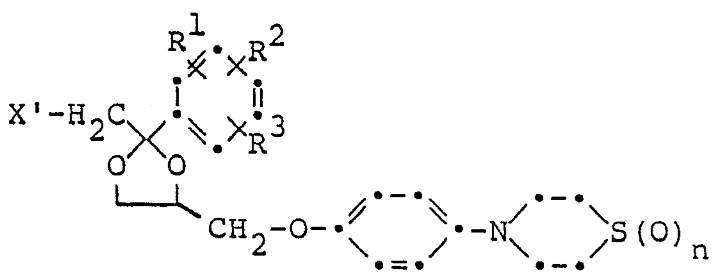

worin $R^1$, $R^2$, $R^3$ und n obige Bedeutung besitzen und X' eine Abgangsgruppe bedeutet, mit einer Verbindung der allgemeinen Formel

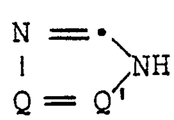

worin Q "und Q' obige Bedeutung besitzen, umsetzt, oder

d) eine Verbindung der allgemeinen Formel

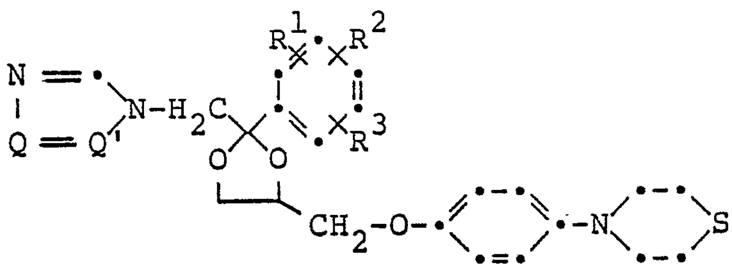

worin $R^1$, $R^2$, $R^3$, Q und Q' obige Bedeutung besitzen, am Schwefelatom oxidiert, und

e) erwünschtenfalls eine Verbindung der Formel I in ein pharmazeutisch annehmbares Säureadditionssalz überführt.

2. Verfahren nach Anspruch 1, dadurch gekennzeichnet, dass Q und Q' beide die Gruppe -CH= bedeuten.

3. Verfahren nach Anspruch 1 oder 2, dadurch gekennzeichnet, dass $R^1$ und $R^2$ je Halogen und $R^3$Wasserstoff bedeuten.

4. Verfahren nach Anspruch 3, dadurch gekennzeichnet, dass $R^1$ und $R^2$ sich in der 2- bzw. 4-Stellung befinden und beide Chlor bedeuten.

5. Verfahren nach einem der Ansprüche 1 bis 4, dadurch gekennzeichnet, dass n die Zahl 2 bedeutet.

6. Verfahren nach Anspruch 1, dadurch gekennzeichnet, dass man cis-4-[4-[[2-(2,4-Dichlorphenyl)-2-[(imidazol-1-yl)-methyl]-1,3-dioxolan-4-yl]methoxy]phenyl]-tetrahydro-4H-1,4-thiazin-1,1-dioxid herstellt.